Europäisches Patentamt

European Patent Office

Office européen des brevets

(19)

(11) Publication number:

**0 306 331**

**A2**

# (12) EUROPEAN PATENT APPLICATION

(21) Application number: **88308154.9**

(22) Date of filing: **02.09.88**

(51) Int. Cl.4: **G 03 G 9/08**

(30) Priority: **02.09.87 JP 221127/87**

(43) Date of publication of application:
**08.03.89 Bulletin 89/10**

(84) Designated Contracting States: **DE FR GB**

(71) Applicant: **SOKEN CHEMICAL & ENGINEERING CO. LTD.,**
**29-5 Takada 3-chome**
**Toshima-Ku Tokyo (JP)**

(72) Inventor: **Imai, Tatsuhiro c/o Soken Chemical & Engineering Co. Ltd. 130 Kamihirose**
**Sayama-shi Saitama (JP)**

**Kawase, Susumu c/o Soken Chemical & Engineering Co. Ltd. 130 Kamihirose**
**Sayama-shi Saitama (JP)**

**Koishi,Masumi c/o Soken Chemical & Engineering Co. Ltd. 130 Kamihirose**
**Sayama-shi Saitama (JP)**

(74) Representative: **Pett, Christopher Phineas et al**
**Frank B.Dehn & Co. Imperial House 15-19 Kingsway**
**London WC2B 6UZ (GB)**

(54) Composite particles and process for preparation thereof.

(57) Composite particles for use in the preparation of super-conductive materials, as fillers for plastics, cosmetics, fillers in chromatography, toners in electrophotography and as pulverulent paints are described. Each particle comprise a spheroidal core, a layer of pulverulent bodies on the surface of said core and a layer of a resin film on the surface of said layer of pulverlent bodies. A process for preparing said particles is also described. The pulverulent bodies do not fall off from the layer of pulverulent bodies. In a case wherein the pulverulent bodies are particulate pigments of dyes, the gloss and color shade of the layer of pulverulent bodies do not deteriorate. Further, the fluidity of the composite particles is not lowered during use and usable kinds of pulverulent bodies are not limited from the view point of their toxicity or safety. In addition the pulverulent bodies are not denatured owing to their hygroscopicity or reactivity with oxygen or the like.

EP 0 306 331 A2

Bundesdruckerei Berlin

**Description**

## COMPOSITE PARTICLES AND PROCESS FOR PREPARATION THEREOF

The present invention relates to composite particles and a process for the preparation of the same. More particularly, it relates to composite particles each comprising a core having pulverulent bodies adhered thereto in which the pulverulent bodies do not break away from the core, do not absorb moisture and are not denatured. It also relates to a process for the preparation of such a composite particle.

Known in the art are composite particles each comprising a spheroidal core having pulverulent bodies, such as particulate pigments and dyes adhered thereto. They are widely used as make-up cosmetics, fillers for plastics and toners for electrophotography.

If the pulverulent bodies such as pigments and dyes are to be adhered to the core while forming a mono-particle layer they may be firmly fixed to the core by hot melting or by embedding them in the core. However, in cases wherein a quantity of pulverulent bodies are attached to the core, they are adhered to the core simply by aggregation, and thus they are likely to break away or fall off from the core by an external force or due to deformation of the core.

The surface of the layer of pulverulent bodies, especially when a quantity of pulverulent bodies such as particulate pigments and dyes are adhered to the core, may gradually become rugged and invite irregular reflection of light, leading to reduction of gloss and/or changes in color shade of the composite particle. Further, as the surface of the layer of pulverulent bodies becomes rugged, the fluidity of the composite particles is lowered.

In a case wherein the prior art composite particle product as described above is to be used in cosmetics, the layer of pulverulent bodies of the product comes in direct contact with skin of the customer, and therefore use of some kinds of pulverulent bodies has had to be avoided for safety reasons. Further, there has also been a problem in that some pulverulent bodies are liable to be denatured during the service life of the composite particles because of their hygroscopicity of reactivity with oxygen or carbon dioxide.

There are still problems associated with the prior art and there remains a need for composite particles each comprising a core having a layer of pulverulent bodies on said core said that the pulverulent bodies do not fall off from the layer of pulverulent bodies.

We have now been able to provide such particles. In the situation wherein the pulverulent bodies are particulate pigments or dyes, the gloss and color shade of the layer of pulverulent bodies of the invention do not deteriorate and the fluidity of the composite particles is not lowered during use. Usable kinds of pulverulent bodies of the invention are not limited from the view point of their toxicity or safety: and the pulverulent bodies of the invention are not denatured owing to their hygroscopicity or reactivity with oxygen or the like. We also provide a process for the preparation of such a composite particles.

Each of the composite particles according to the invention comprises a spheroidal core, a layer of pulverulent bodies on the surface of said core and a layer of a resin film on the surface of said layer of pulverulent bodies.

The process for the preparation of composite particles according to the invention comprises the steps of adhering pulverulent bodies to the surface of each spheroidal core, microcapsulating said pulverulent bodies to form a layer on the surface of each said core, adhering particles of a resin on the surface of said layer of pulverulent bodies and mirocapsulating said particles of resin to form a layer of a resin film on the surface of said layer of pulverulent bodies on each said core.

Since the composite particle according to the invention comprises a spheroidal core, a layer of pulverulent bodies on the surface of said core and a layer of a resin film on the surface of said layer of pulverulent bodies, the characteristics set out above are obtained.

The composite particles and process for the preparation thereof according to the invention will now be described in detail by way of example with reference to the attached drawings, in which:

Fig 1 is a diagrammatic cross-sectional view of a composite particle according to the invention; and

Figs. 2 and 3 are diagrammatic cross-sectional showings for illustrating the steps of the process according to the invention.

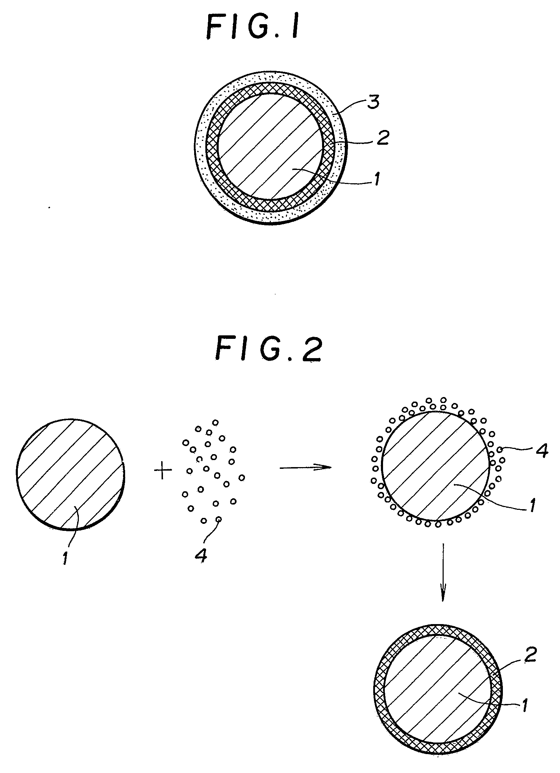

As shown in Fig. 1, the composite particle according to the invention comprises a spheroidal core 1, a layer 2 of pulverulent bodies on the surface of said core 1 and a layer 3 of a resin film on the surface of said layer 2 of pulverulent bodies.

The spheroidal core 1 may be formed from thermoplastic resins, thermosetting resins, pigments, dyes, metals, ceramics, naturally occurring organic high polymers, inorganics and carbonized resins. The thermoplastic resins which can be used to form the spheroidal core 1 include styrene resins, acrylics, polyolefin resins such as polyethylene and polypropylene, nylons and other polyamide resins, silicone resins, fluorine resins and polyester resins. The thermosetting resins which can be used to form the spheroidal core 1 include epoxy resins and phenolic resins. Inorganic cores comprising glass or silica, naturally occurring organic high polymer cores comprising cellulose or starch, metallic cores comprising ferrite or zinc oxide, and ceramic cores comprising alumina or titania, may also be used herein.

The individual spheroidal cores 1 should preferably have approximately the same particle size ranging between 0.5 and 200 $\mu$m, preferably between 1 and 10 $\mu$m.

The spheroidal core 1 is provided with a layer 2 of pulverulent bodies on its surface. The pulverulent bodies 4 which comprise the layer 2 may be pigments or dyes.Specifically, as red pulverulent bodies inorganic pigments such as iron oxide red and cadmium red, organic pigments such as quinacridone red, Brilliant red, Karmine 6B and azo red, and dyeable lake pigments; as blue pulverulent bodies, inorganic pigments such as prussian blue, ultramarine blue and cobalt blue, organic pigments such as phthalocyanine blue and indigo blue, and dyeable lake pigments; and as yellow pulverulent bodies, inorganic pigments such as titanium yellow, yellow lead and iron oxide yellow. organic pigments such as azo yellow, isoindolinone yellow and fast yellow, and dyeable lake pigments can be used. In a case wherein a metallic colored composite particle is desired, metallic pigments such as aluminum, bronze, gold, silver, nickel and brass, can be used herein.

In some cases, the layer 2 may be composed of photochromic, pharmaceutical, or agriculturally pesticidal pulverulent bodies, proteins or enzymes.

The pulverulent bodies used, prior to being formed to the layer 2, are preferably particulate and have a primary particle size of from 0.01 to 2 μm, preferably, from 0.02 to 1.0 μm.

The thickness of the layer 2 is determined in accordance with the desired color shade of the composite particles, and the thicker the layer 2 the deeper the color shade of the composite particles. Normally the thickness of the layer 2 of pulverulent bodies is within the range from 0.05 to 2 μm.

The layer 2 of pulverulent bodies is provided with a layer 3 of a resin film. The layer 3 is formed of a thermoplastic or thermosetting resin. The thermoplastic resins which can be used to form the layer 3 include styrene resins, acrylics, polyolefin resin such as polyethylene and polypropylene, nylons and other polyamide resins, silicone resins, fluorine resins and polyester resins. The thermosetting resins which can be used to form the layer 3 include epoxy resins and phenolic resins.

The layer 3 of a resin film is formed, as described hereinafter in detail, by adhering resin particles to the surface of the layer 2 formed on the spheroidal core 1, and microcapsulating the resin particles to the layer 3, for example, by subjecting the same to a impact treatment in a gaseous flow to flatten the resin particles to a film. The particles of the resin used herein are fine particles of thermoplastic or thermosetting resins which have preferably been finely divided by e.g. a jet mill. The particle size of the resin is normally from 0.05 to 5 μm, preferably from 0.1 to 1 μm, and is normally not larger than one fifth, preferably not larger than one tenth of the particle size of the spheroidal core 1.

The resin particles used may be statically charged. By using such charged resin particles, the state of static electrification of the obtainable composite particles may be controlled.

In order that the state of static electrification of the composite particle according to the invention is substantially determined by that of the outer most layer of resin, resin particles charged to an appropriate extent in accordance with the desired level of static electrification of the composite particles are used to form the outermost layer 3. For this purpose the resin particles used preferably have an absolute amount of static electrification larger than, preferably at least 2 times, and more preferably at least 3 times that of the spheroidal core 1. More specifically, the absolute amount of static electrification of the resin particles for forming the layer 3 is desirably at least 50 μC/g.

The amount of static electrification of the resin particles can be controlled or modified by surface treatment thereof as described in "Technologies of Surface Reforming and Improvement of Surface Functions of Particulate Bodies", SURFACE, Vol. 25, No. 1 (1987). Alternatively, the amount of static electrification of the resin particles can be controlled or modified by introducing polar groups into the resin particles upon manufacture thereof.

In the process for the preparation of composite particles according to the invention, as shown in Fig. 2, pulverulent bodies 4 are first adhered to the surface of each of spheroidal cores 1, and mirocapsulated to form a layer 2 of pulverulent bodies on the surface of each spheroidal core 1.

The adhesion of the pulverulent bodies 4 to the individual cores 1 can be conveniently done by dry blending the cores 1 with the pulverulent bodies 4 whereupon the cores 1 are frictionally charged and attract the pulverulent bodies 4.

The pulverulent bodies 4 adhered to the individual cores 1 are then microcapsulated to form layers 2 on the individual cores 1 by an impact treatment of the cores 1 having the pulverulent bodies 4 adhered under a gaseous flow. The impact treatment may comprise bringing the cores 1 to collide to each other or applying a mechanical impact to the cores 1.

This microcapsulation can be conveniently done using a commerically available apparatus for dry reforming surfaces of particulate bodies, NHS-1, supplied by NARA Machinery Co., Ltd.

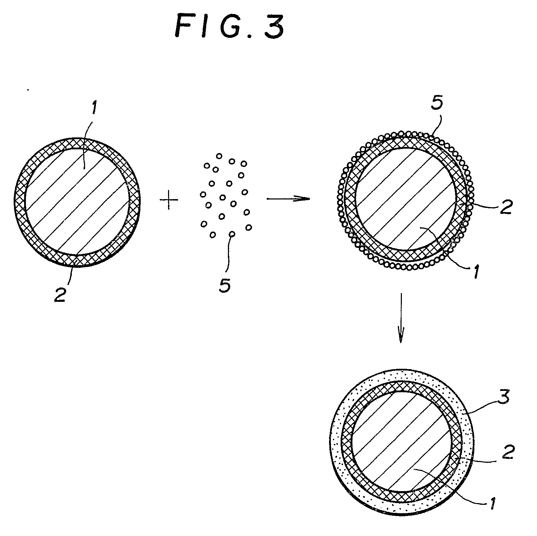

In the process for the preparation of a composite particle according to the invention, as shown in Fig. 3, resin particles 5 are adhered to the surface of the layer 2 of pulverulent bodies on each spheroidal core 1 and then microcapsulated to form the outermost layer 3 of a resin film on the layer 2 of pulverulent bodies.

The adhesion of the resin particles 5 to the pulverulent layer 2 of the individual cores 1 can be conveniently done by dry blending the cores 1 having the pluverulent layer 2 with the resin particles 4 and the microcapsulation of the resin particles 5 can be conveniently done by an impact treatment of the cores 1 having the resin particles 5 adhered thereto via the pulverulent layer 2 under a gaseous flow. Examples of the gas, which can be used, include,. for example, air, catbon dioxide, nitrogen, argon and other inert gases. The impact treatment may comprise bringing the cores 1 to collide to each other or applying a mechanical impact to the cores 1.

In the situation wherein positively or negatively charged resin particles are used to form the layer 3 of resin film, composite particles having a controlled state of static electrification may be obtained in addition to the advantages set forth above.

Intended uses of composite particles according to the invention include, for example, cosmetics, pulverulent bodies for use in the preparation of superconductive materials, fillers for plastics, fillers for use in chromatography, toners for use in electrophotography and pulverulent paints. In a case of cosmetics comprising composite particles according to the invention, they are excellent in that since each particle has an outermost layer of a resin film, they pose no problems on toxicity; and the pulverulent bodies used do not absorb moisture, are not deteriorated by ultraviolet ray, are not denatured by reacting with oxygen or carbon dioxide, and do not fall off from the composite particles.

In a case of a toner for use in electrophotography comprising composite particles according to the invention the state of static electrification of the toner can be precisely controlled by simply controlling the amount of static electrification of the resin particles used to form the outermost layer.

The invention will now be further described by the following examples, which should not be considered as limiting:

## Example 1

150 grams of polymethyl methacrylate particles having a relatively uniform particle size of 5 μm and 50 grams of a phthalocyanine blue, Blue No. 404,, were treated in an apparatus for dry reforming surfaces of particulate bodies (NHS-1, supplied by NARA Machinery Co., Lid.) for a period of 5 minutes to provide colored particles (I) surface coated with phthalocyanine blue.

To 180 grams of the thus-obtained colored particles (I) surface coated with phthalocyanine blue, 40 grams of polymethyl methacrylate powder having a particle diameter of 0.4 μm were adhered and treated in an apparatus for dry reforming surfaces of particulate bodies of the same type as mentioned above for a period of 8 minutes to provide particles (II) surface coated with polymethyl methacrylate. The particles (II) so obtained contained about 20 % by weight of phthalocyanine blue.

## Example 2

180 grams of polystyrene particles having a relatively uniform particle size of 8 μm and 20 grams of a rancid enzyme lypase were treated in an apparatus for dry reforming surfaces of particulate bodies (NHS-1, supplied by NARA Machinery Co., Lid.) for a period of 5 minutes to provide particles (III) surface coated with rancid enzyme lypase.

To 160 grams of the so obtained particles (III) surface coated with rancid enzyme lypase, 40 grams of polybutyl methacrylate powder having a particle diameter of 0.4 μm were adhered and treated in an apparatus for dry reforming surfaces of particulate bodies of the same type as mentioned above for a period of 5 minutes to provide particles (IV) surface coated with polybutyl methacrylate. The particles (IV) so obtained contained about 8 % by weight of rancid lypase.

## Example 3

150 grams of polystyrene gel particles having a relatively uniform particle size of from 10 to 30 μm and 50 grams of aluminum powder were treated in an apparatus for dry reforming surfaces of particulate bodies (NHS-1, supplied by NARA Machinery Co., Lid.) for a period of 5 minutes to provide particles (V) surface coated with aluminum.

To 100 grams of the so obtained particles (V) surface coated with aluminum 40 grams of polymethyl methacrylate powder having a particle diameter of 0.4 μm were adhered and treated in an apparatus for dry reforming surfaces of particulate bodies of the same type as mentioned above for a period of 8 minutes to provide particles (VI) surface coated with polymethyl methacrylate. The particles (VI) so obtained contained about 20 % by weight of aluminum.

The particles (VI) had better gloss than those products having an aluminum coat formed by plating or vacuum evaporation.

An SCM resin composition having 6 % by weight of the particles (VI) based on the resin was molded under conditions of a temperature of 140 °C., a pressure of 10 kg/cm$^2$ and a reaction time of 3 minute. An SCM molding with a uniform metallic gloss having transparent appearance was obtained.

## Example 4

250 grams of glass beads having a particle size ranging from 30 to 70 μm and 50 grams of titanium yellow were treated in an apparatus for dry reforming surfaces of particulate bodies (NHS-1, supplied by NARA Machinery Co., Lid.) for a period of 5 minutes to provide colored particles (VII) surface coated with titanium yellow.

To 180 grams of the so obtained colored particles (VII) surface coated with titanium yellow, 20 grams of polytetrafluoroethylene powder having a particle diameter of 0.3 μm were adhered and treated in an apparatus for dry reforming surfaces of particulate bodies of the same type as mentioned above for a period of 8 minutes to provide particles (VIII) surface coated with polytetrafluoroethylene.. The particles (VIII) so obtained contained about 15 % by weight of titanium yellow.

The particles (VIII) and an acrylate modified silicone resin (Thermolac H-301-SE) were mixed together in a weight ratio of 20 : 80, whereupon the particles rapidly dispersed into the resin and the resin layer became transparent. The paint so prepared was applied to a glass plate and cured under heat. A coating having a transparent appearance was obtained.

## Example 5

150 grams of spheroidal particles of a copolymer of styrene and butyl acrylate having a particle size of ranging from 5 to 15 μm and 50 grams of carbon black were treated in an apparatus for dry reforming surfaces of particulate bodies (NHS-1, supplied by NARA Machinery Co., Lid.) for a period of 5 minutes to provide colored particles (IX) surface coated with carbon black.

To 160 grams of the so obtained colored particles (IX) surface coated with carbon black, 40 grams of positively charged polymethyl methacrylate powder having a particle diameter of 0.25 μm were adhered and treated in an apparatus for dry reforming surfaces of particulate bodies of the same type as mentioned above for a period of 8 minutes to provide (X) surface coated with polymethyl methacrylate. The particles (X) so obtained had a blow-off charge of 30 μC/g.

When compared with the particles (IX), the toner particles (X) had an improved fluidity and did not undergo blocking during storage.

Example 6

160 grams of spheroidal particles of a phenolic resin having a particle size of ranging from 5 to 10 μm and 40 grams of Kermine red were treated in an apparatus for dry reforming surfaces of particulate bodies (NHS-1, supplied by NARA Machinery Co., Lid.) for a period of 5 minutes to provide colored particles (XI) surface coated with Kermine red.

To 170 grams of the so obtained particles (XI) surface coated with Kermine red, 30 grams of vinylidene fluoride resin powder having a particle diameter of 0.3 μm were added and treated in an apparatus for dry reforming surfaces of particulate bodies of the same type as mentioned above for a period of 4 minutes to provide particles (XII) surface coated with vinylidene fluoride resin. When compared with the particles (XI), the particles (XII) had remarkably improved water and oil repellency and static electrification property, although the fluidity of particles (XII) was similar to that of the particles (XI)

Example 7

160 grams of spheroidal beads of a carbonized phenolic resin having a particle size of from 10 to 30 μm and 40 grams of a phthalocyanine blue were treated in an apparatus for dry reforming surfaces of particulate bodies (NHS-1, supplied by NARA Machinery Co., Lid.) for a period of 4 minutes to provide colored particles (XIII) surface coated with phthalocyanine blue. To 180 grams of the so obtained particles (XIII) surface coated with phthalocyanine blue 20 grams of polymethyl methacrylate powder having a particle diameter of 0.8 μm were adhered and treated in an apparatus for dry reforming surfaces of particulate bodies of the same type as mentioned above for a period of 5 minutes to provide particles (XIV) surface coated with polymethyl methacrylate. Examination of the particles (XIII) and (XIV) by means of color differential meter revealed that the particles (XIV) had a deeper color shade than the particles (XIII).

Claims

1. Composite particles each comprising a spheroidal core, a layer of pulverulent bodies on the surface of said core and a layer of a resin film on the surface of said layer of pulverulent bodies.

2. Particles as claimed in claim 1 wherein the core diameter is between 0.5 and 200 μm, preferably between 1 and 10 μm.

3. Particles as claimed in claim 1 or claim 2 wherein the pulverulent bodies on the surface of said core have a primary particle size of 0.01 to 2μm, preferably from 0.002 to 1.0μm.

4. Particles as claimed in any of claims 1 to 3 wherein the thickness of the layer of pulverulent bodies is from 0.05 to 2μm.

5. Particles as claimed in any of claims 1 to 4 wherein the particle size of the resin is from 0.05 to 5μm, preferably from 0.1 to 1μm and does not exceed a fifth of the particle size of the spheroidal core.

6. Particles as claimed in any of claims 1 to 5 wherein the resin particles are statically charged.

7. A process for the preparation of a composite particle comprising the step s of adhering pulverulent bodies to the surface of a spheroidal core, microcapsulating said pulverulent bodies to form a layer on the surface of said core, adhering particles of a resin on the surface of said layer of pulverulent bodies, and microcapsulating said particles of resin to form a layer of a resin film on the surface of said layer of pulverulent bodies on said core.

# FIG.1

# FIG.2

# FIG. 3